# EUROPEAN PATENT APPLICATION

(11) **EP 4 012 407 A1**
(43) Date of publication of application: **15.06.2022**
(21) Application number: 20213960.6
(22) Date of filing: 14.12.2020
(51) Int. Cl.: G01N 33/49, G01N 35/00

(54) **SETTING CLOTTING TIME FOR BLOOD SAMPLES**

(71) Applicant: Beckman Coulter Inc., Brea, California 92821 (US)
(72) Inventor: TICHIT, Didier, 81375 Munich (DE); LANVIN, Dany, 34680 Saint Georges D'Orques (FR)
(74) Representative: Vos, Derk

(57) **Abstract**

Embodiments of the present disclosure relate to a method, system and apparatus for setting a clotting time for a blood sample contained in a container in a laboratory device or test system, which includes setting a clotting start time for the blood sample, setting a clotting wait time to allow for the blood sample to clot, and on positive determination of the completion of the clotting wait time, automatically providing the blood sample for further processing in the laboratory.

## Description

### TECHNICAL FILED

This disclosure generally relates to the field of clinical diagnosis and/or laboratory systems. In particular, the present disclosure relates to a method of processing a blood sample contained in a container in a test system. Further, the disclosure relates to a laboratory test system configured to perform said method and to a computer-readable medium comprising instructions to instruct a laboratory test system to perform said method.

### BACKGROUND

Typically, blood clotting is a complex process that may involve three interacting components: blood vessels, blood coagulation factors, and blood platelets. Usually, blood coagulation factors such as proteins or glycoproteins freely circulate within the body. Generally, blood coagulation factors interact in a mechanism commonly referred to as a coagulation cascade.

Chemistry and Immunoassay analyzers typically use serum as a substrate for analysis. To obtain serum, a blood sample is usually allowed to clot (coagulate) in a sample container and is then separated into the solid and the liquid fraction, normally via centrifugation of the sample container. The resulting liquid supernatant is referred to as a serum. Though several methods for accelerating and delaying the clotting process of blood may be known, there may be a need for a process of ensuring that the blood sample provided to an analyzer (also referred to as laboratory instrument, laboratory device or test station) for processing has been properly coagulated, prior to performing a respective analysis of the serum on the analyzer. A disadvantage in the clotting process is that if for the blood the clotting process has not been completed properly, latent fibrin formation in the resultant serum could cause the analyzer to malfunction and may even result in providing incorrect results for samples to be processed on the analyzer.

It may therefore be an object of the present disclosure to ameliorate one or more disadvantages known in the prior art. Particularly, the present disclosure provides for an improved method, apparatus, system, and computer program product.

### SUMMARY

Aspects of the present disclosure relate to a method, system, apparatus and computer program product for setting a clotting time for a blood sample contained in a container in a test system (hereinafter also referred to as a laboratory device or laboratory test system), which includes setting a clotting start time for the blood sample, for example subsequent to setting a clotting start time, setting a clotting wait time to allow for the blood sample to clot, and finally on positive determination of the clotting wait time to have been completed, automatically providing the blood sample to a centrifuge and/or triggering testing and/or transportation to a centrifuge.

It should be noted that any feature, function, step and/or element described hereinabove and hereinbelow with respect one aspect of the present disclosure, equally applies to any other aspect of the present disclosure. Specifically, any step, feature, function, and/or element described hereinabove and hereinbelow with respect to the method equally applies to the (laboratory) test system, and vice versa.

The invention is defined in the appended independent claims, wherein further aspects and embodiments are also disclosed in the following description, the figures and the dependent claims.

According to a first aspect of the present disclosure, there is provided a method for processing a blood sample contained in a container in a test system. The test system may also be referred to hereinafter as laboratory test system, system, laboratory system, apparatus, or laboratory device. The method according to the first aspect may be partly or entirely computer-implemented and/or computer-assisted. Alternatively or additionally, the method may refer to a method of operating a test system to process a blood sample contained in a container. The method comprises setting a clotting start time for the blood sample and/or the container, and setting a clotting wait time to allow the blood sample to clot. Therein, the clotting start time may be set before, after or simultaneously to setting the clotting wait time. Further, the method comprises a step of, upon positive determination of completion of the clotting wait time, automatically providing the blood sample and/or the container to a centrifuge of the test system and/or coupled to the test system. Alternatively or additionally, the container can be provided to a test station and/or an analyzer of the test system (and/or coupled to the test system).

Therein, providing the blood sample to the centrifuge, test station and/or the analyzer may include triggering transport of the container, e.g. based on providing, for example by a controller, control circuitry, or processor of the test system, a corresponding control signal to a conveyance system. Alternatively or additionally, providing the blood sample may include physically transporting, e.g. by a conveyance system, the container to the centrifuge, test station and/or the analyzer in a at least partly or fully automated manner. In the context of the present disclosure, "automatically" or "automated" may refer to or include computer-controlled and/or computer-assisted.

Generally, the method of the present according to the first aspect advantageously allows to ensure an adequate clotting wait time to allow for sufficient clotting, for example minimizing, reducing or eliminating latent fibrin in the serum. Accordingly, the method of the first aspect can ensure reliable test results.

As noted above, aspects of the present disclosure are related to a method, system, apparatus and computer program product for processing a blood sample contained in a container in a test system, by e.g. first setting and/or recording and/or logging a clotting start time for the blood sample, e.g. then setting and/or fixing and/or adjusting a clotting wait time to allow the blood sample to clot, and on positive determination of completion of the clotting wait time, automatically providing the blood sample to a centrifuge for further processing. Adjusting of the clotting wait time can be manually performed by the clinician, specifically when the manufacturer has set a clotting wait time for the container and there may optionally be other additional pre-determined factors mentioned above that could potentially influence the clotting wait time. For example the manufacturer may have suggested that the clotting wait time from the container be 20 mins, but the patient may be on an coagulant medication, which in turn may increase the clotting wait time, for example the clotting wait time may be manually increased or the clotting wait time may be suitably adjusted by adding of a clotting agent to the blood sample.

In the context of the present disclosure, the clotting start time may refer to or denote a time instant or a time period. Likewise, the clotting wait time may refer to or denote a time instant or time period. The same applies to any other "time" referred hereinabove and hereinbelow.

In one embodiment the clotting start time may be set as the time of withdrawal of the blood sample into the container. The time of withdrawal of the blood sample into the container may for example be obtained from a LIS (Lab information system) or HIS (Hospital information system) or any other relevant data source, for example in the simplest form a sample bar code encoding the time of withdrawal of the blood sample may be placed on the container with other relevant data/information.

In another embodiment, the clotting start time may be set as the time of loading the sample in the container onto the laboratory device. A time of loading the container containing the blood sample onto the test system may be manually entered by a user of the test system or may be automatically set by the test system as the time when the test system first recognizes the presence of the blood sample within the test system, for example by detecting from an information tag or identifier, such as the sample bar code, which includes container related information. The test system may be configured to read such identifiers on a container, which may include a printed bar core pasted on the container, RFID tag or the like.

In a further embodiment, the clotting wait time may be determined based the time of withdrawal of the blood sample into the container. The time of withdrawal of the blood sample may be recorded on the container on an identifier such as a bar code and the test system is configured to read such a bar code.

In a further embodiment the clotting wait time may be determined based on the time of loading the sample in the container onto the test system. Again, the time of loading the sample on the test system may be further recorded on the container on the bar code.

In a further embodiment, the clotting wait time may be determined based on the addition of at least one clotting agent to the blood sample. For example if the patient is on an anti-coagulant medication, the addition of a clotting agent to the blood sample may accelerate the clotting process.

In a further embodiment, a storage temperature of the blood sample in the storage unit after collection of the blood sample may be considered by the test system to adjust the clotting wait time. Normally clotting would occur at a room temperature, 25 to 30 degrees, in a time period of 2- - 30 minutes, however, if the storage is maintained at a lower temperature, the clotting process may be delayed accordingly.

The temperature may for example be considered based on receiving and/or processing temperature data indicative of a temperature of the container and the blood sample. Such temperature data may be received from a sensor and/or acquired by a sensor. Alternatively or additionally, temperature data may be provided based on a user input, e.g. at a user interface of the test system.

In a further embodiment, the clotting wait time may be adjusted based on and/or considering a measure of an air pressure and/or a measure of humidity during a storage of the blood sample. Just as temperature may influence the clotting process, air pressure and humidity may also result in delaying or accelerating the clotting process for the blood sample.

In a further embodiment, at least one patient specific coagulation parameter may be considered for determining the clotting wait time. For example if the patient's EHR indicated that the patient is on anti-coagulant medication, such a medication may delay the clotting process for the blood sample, and in such cases it may be advantageous to identify and add a clotting agent to accelerate the clotting process.

In a further embodiment, the clotting wait time may be determined and/or adjusted based on a predefined clotting wait time, e.g. as specified by the test system manufacturer and/or stored in a data storage of the test system. For example, if the manufacturer has coated the container tubes with silica, then the manufacturer may specify a quicker clotting wait time as silica inherently acts as a clotting agent for the blood sample and results in faster clotting of the blood sample.

According to an embodiment, the clotting wait time is determined based on at least one of a time of withdrawal of the blood sample into the container, a time of loading the blood sample in the container onto the test system, addition of at least one clotting agent to the blood sample and/or the container, a storage temperature of the blood sample, a measure of an air pressure and/or a measure of humidity during a storage of the blood sample, at least one patient specific coagulation parameter, a predefined clotting wait time, e.g. as specified by the test system manufacturer.

According to the present disclosure, the clotting wait time for a specific blood sample may be determined such that, counting from the clotting start time, the clotting reaction within the specific blood sample may be basically or substantially completed, when the clotting wait time elapses. The clotting start time may be selected as the time of withdrawal of the blood sample into the container and/or may be the time of loading the sample in the container onto the test system and/or may be the time when the container was placed into a storage rack in a vertical position, and/or may be another point in time which may be suitable to indicate the beginning of the blood clotting reaction.

The clotting wait time may be initially set as a default time span or period, which may be for example a time span of 30 min, 45 min or 60 min. Subject to the availability of one or more additional blood sample related data, the clotting wait time may be modified, computed and/or adjusted, e.g. by a controller, control circuitry and/or processor of the test system.

For example, if the additional blood sample related data includes information about the addition of at least one clotting agent to the blood sample, the clotting wait time may be reduced accordingly. For example, spontaneous and complete clotting of blood samples normally occurs within a period of 30 to 60 minutes at room temperature (20 to 25 degree centigrade). Alternatively, if the additional blood sample related data includes information about blood sample having been stored below room temperature after the blood was drawn, the clotting wait time can be extended accordingly, as it may take longer for clotting to occur at lower temperatures. Again, for example, the blood sample may be drawn at room temperature and the storage and transportation of the collected blood sample may be at a lower temperature, such as between 2 to 8 degree centigrade, which may generally result in a delay for clotting of the blood sample. Alternatively, if the additional blood sample related data includes information about blood sample having been stored above room temperature, for example at a higher temperature, after the blood was drawn, the clotting wait time can be reduced accordingly. For example, the blood sample may be drawn at room temperature and the storage and transportation of the collected blood sample may be at a higher temperature, such as between 30 to 35 degree centigrade, which may generally result in a faster clotting of the blood sample.

Again, for example, spontaneous and complete clotting of blood samples normally occurs within a period of 30 to 60 minutes at room temperature (20 to 25 degree centigrade). However, if the humidity in the storage is higher than normal, the clotting may occur faster and if the humidity in the storage is lower than normal then clotting may be delayed, and in such case the clotting wait time may be accordingly adjusted. In a further embodiment at least one patient specific coagulation parameter may be derived from an electronic medical record (EMR) or a Laboratory Information System, or a Hospital Information System or any other Personal Health Information that may be made available to the laboratory. Based on the patient history available via an EMR, which indicates that the patient is on anti-coagulant medication, a clotting agent may be added to the blood sample to accelerate the clotting process. If for example, a patient EMR indicates that the patient is on a specific anti-coagulation medication, a clotting agent may be added to the blood sample immediately after withdrawal of the blood sample from the patient to accelerate the clotting process at the clotting station or alternatively the clotting wait time may be increased manually by means of user interface coupled to the test system, so that further processing of the blood sample occurs after the clotting wait time has elapsed. Alternatively, if a patient's EMR indicated that the patients' blood clots very quickly, the clotting wait time for the blood sample can be reduced manually by means of user interface.

In a further embodiment, a predefined clotting wait time may be specified by the manufacturer of the test system or the supplier of a sample collection containers. The predefined clotting wait time may be stored in a data storage of the test system. The manufacturer may also specify for example specific clotting wait time periods corresponding to a variety of clotting/coagulation agents or anti-coagulation agents that may be present in a sample tube. For example, if silica particles are used in the sample collection containers, the clotting wait time may be reduced as compared to standard samples, for example to a period of 15 to 20 minutes. It should be noted here that two or more of these factors discussed above can be combined to either increase or decrease the clotting wait time for the blood sample.

According to an embodiment, the clotting wait time is entered, received and/or provided via a user interface of the test system and/or coupled to the test system.

According to an embodiment, the clotting wait time is selected, set, computed and/or determined automatically by the test system.

According to an embodiment, the clotting wait time is adjusted to be either longer than or shorter than a predefined default clotting wait time based on the at least one patient specific coagulation parameter.

In a further embodiment a timer for monitoring the clotting wait time may be included or built into the test system. For example, the timer may be internal (in-built) into the test system or external to the test system. For example, a CPU clock can be used as an internal timer for monitoring the clotting wait time. An external timer for monitoring the clotting wait time can be realized by any software or hardware that may have an appropriated interface to the test system, for example a middleware software.

In a further embodiment the container containing the blood sample may be subjected to a centrifugation step in order to segregate the serum from the clotted blood sample. On positive determination of the clotting wait time to have elapsed, the container may be automatically or manually provided to a centrifuge and then subjected to the process of centrifugation to separate the serum from the clot. The centrifuged sample container may then be provided to at least one test station (also referred to herein as analyser) of the test system for further analysis. The analyzer(s) may be either connected via a sample transport system of the test system or, if stand-alone analyzer(s) are used, the centrifuged samples can be transported manually from the test system to such analyzers. In a further embodiment a test station may be a blood serum analyzer.

In a further embodiment a clotting agent may be included or added to the blood sample, wherein the clotting agent is configured to reduce the clotting wait time of the blood sample.

In a further embodiment the clotting wait time may be entered via a user interface coupled to the test system depending on various parameters, such as patient specific data, for example data obtained from an EMR, sample storage temperature etc., as discussed previously and below. The clotting wait time for at least one sample may be automatically calculated and used by the test system based on one or more available blood sample specific coagulation or anti-coagulation parameters. Alternatively or additionally, the test system may allow the user to modify the proposed automatically calculated clotting wait time for one or more samples. As a guidance for such modification the system may display to the user one or more factors, that may have been taken into account for the calculation of the proposed clotting wait time. If pre-defined clotting time values are used, these may e.g. be specified by the manufacturer of the test system or via user input, any software run on the test system and/or the user of the test/system.

In a further embodiment the clotting wait time may be adjusted to be either longer than or shorter than a predefined default clotting wait time based on the at least one patient specific coagulation parameter. For example, if the patient is on anti-coagulation medication, a coagulation/clotting agent may be used to accelerate the clotting reaction within the sample. Both the anti-coagulation medication and the used coagulation agent may then be taken into account for calculating the clotting wait time. If a clotting agent is not used, then the clotting wait time may be calculated based on the anti-coagulation medication that is used by the patient, and the clotting wait time may be manually increased by the clinician from the default values set therein.

In a further embodiment the container may be preferably held in a vertical position during the clotting wait time, wherein the top of the container is either open or closed with a cap, a lid, a stopper, a film, or any other container closing structure. This ensures that clotting does not happen in the area of the container closing structure and also prevents spillage of sample liquid when opening the contained and a possible contamination of the serum sample with remnant fibrin when providing the sample to the analyzers of the test system.

In another embodiment, the time of the withdrawal of the blood sample into the container is obtained from a sample related data source. The sample related data source may be any database, laboratory or hospital information system, a middleware software or any other data repository which comprises information about the time of the blood sample draw. The blood sample draw time for a specific sample may be obtained by reading a sample container tag, coupled to the specific sample container. Such a container tag is considered to constitute another kind of sample related data source. The sample container tag may either directly include the information about the blood sample draw time, or it may comprise a sample ID, which can be used to search said data repository for the sample specific blood sample draw time. The sample container tag may for example be read optically or electronically.

In a further embodiment the container includes, as a sample related data source, a unique identifier comprising at least one of a bar code, a MEMS, smart dust, an RFID chip or a NFC chip coupled to the container. This unique identifier may be encoded with a patient ID, patient details, tests required, a time stamp etc.. The test system may use the time stamp as the clotting start time in order to calculate the clotting wait time. Each container may be associated with a unique tag or identifier, and may further include information relating to the sample's blood draw time or other data which can be used for calculating the clotting start time.

According to an embodiment, the at least one patient specific coagulation parameter is derived from and/or received from at least one of an electronic medical record (EMR), a Laboratory Information System (LIS), a Hospital Information System (HIS), and any other Personal Health Information (PHI).

A second aspect of the present disclosure relates to a test system including a container storage for storing the at least one container containing the blood sample for the duration of the clotting wait time and an automated sample conveyance system (also referred to as a conveyance unit) coupled to one or more test stations, preferably one or more at least partly automated test stations. The test system is configured to set a clotting start time, determine a clotting wait time, for example based on one or more (pre-defined parameters) as described hereinabove and hereinbelow, and upon positive determination of the clotting wait time to have elapsed, processing the blood sample for further analysis.

The conveyance system may be configured to move sample containers within one or more modules of the test system, including one or more test stations or analyzers connected to the test system, depending on the type of test to be performed on the blood sample.

The system according to the second aspect of the present disclosure may particularly be configured to perform one or more steps of the method of the first aspect.

### BRIEF DESCRIPTION OF THE DRAWINGS

Embodiments of the present disclosure are illustrated by way of example and not limitation in the figures of the accompanying drawings.
FIG 1 illustrates an exemplary embodiment of a biological sample test system;
FIG. 1A illustrates an exemplary graphical user interphase illustrating clotting time overview for multiple samples;
FIG 2 illustrates an exemplary embodiment of a method for processing a blood sample according to the disclosure; and
FIG. 3 illustrates an exemplary embodiment of a block diagram illustrating components of a machine able to read instructions from a machine-readable medium.

### DETAILED DESCRIPTION OF EXEMPLARY EMBODIMENTS

Exemplary methods and systems of the present disclosure are directed to specifically to processing of blood samples in a diagnostic instrument, a laboratory system, an analyzer, a laboratory device, or the like. Examples disclosed herein are merely illustrative in nature and there could be possible typified variations. Unless explicitly stated otherwise, components and functions are optional and may be combined or subdivided, and operations may vary in sequence or be combined or subdivided. In the following description, for purpose of explanation and simplicity, numerous specific details may be set forth to provide an understanding of the examples and disclosed embodiments.

The disclosure may be considered as being based on the following insights and findings. To create a serum for a serum analysis, generally a container or a vial of blood collected from a patient may be allowed to clot or coagulate. A resultant liquid after the clotting of the blood sample has occurred is normally referred to as a serum. Creation of a serum may generally depend on the creation of a suitable clot. Normally, creation of a clot may be based on a variety of factors, including but not limited to holding the container still in a predetermined orientation, preferably in a vertical orientation with the cap on the top of the container. Typically, creation of a clot may also depend on specific environmental condition, such as temperature and/or pressure and/or humidity for a predetermined period of time while the blood sample is stored in a storage for clotting to occur. A variance of any of these conditions may generally result in an unsuitable clot for the creation of serum, which may lead to completely undermining subsequent testing of the blood sample, for example due to the presence of latent fibrin being present in the serum.

In accordance with the embodiments of the present disclosure, a clotting time setting method, system and a computer program product has been designed for a test system (also referred to as a laboratory device or laboratory system), which includes preparing of a serum from the blood sample for serum analysis. In a further embodiment, the computer program product includes either a software or a hardware or a combination thereof, interfaced with or being part of the test system to execute a set of instructions to be performed on the test system. In a further embodiment, a clotting verification method, system and computer program product may include a user interface for a user, for example a clinician or technician, to manually input the clotting time for a particular sample, wherein the user may be able to enter a default value, which may be set as a minimal value of clotting time, that may be observed for all samples, unless there are other factors influencing the clotting time, for example patient specific parameters that may lead to an extension or reduction of the clotting wait time. In a further embodiment, upon withdrawal of the blood from a patient (also referred to broadly as a user) into a container, the container possessing the blood sample may be stamped with the Patient ID and a time stamp of collection of the blood sample. In a further embodiment a timer may be activated upon receipt of a container containing the blood sample, for example the timer may be a clock associated with or incorporated into the test system. The timer may be set for a pre-determined amount of time that may be considered as the time required for a sufficiently complete clotting reaction to produce a serum fraction and a clot. The time for clotting to occur may vary depending on various factors. For example, spontaneous and complete clotting of blood samples normally occurs within a period of 30 to 60 minutes at room temperature (20 to 25 degree centigrade). For example, the blood sample may be drawn at room temperature and the storage and transportation of the collected blood sample may be at a lower temperature, such as between 2 to 8 degree centigrade, which may generally result in a delay of clotting of the blood sample.

Generally, if the time allowed for clotting is inadequate, latent fibrin may remain in the serum, which may then cause a problem for many analyzers (also referred to as diagnostic instruments or laboratory instruments). Usually, tube or container manufacturers may provide recommendations to ensure that proper clotting of the sample occurs. For example, the container manufacturer may suggest a faster clotting wait time because of the coating the containers with silica, which acts as a clotting agent. Further, a user interface, for example on the test system, may indicate whether the clotting wait time for a container with a sample has already elapsed or alternatively indicate the remaining time required for the clotting reaction to occur. When the clotting wait time has elapsed, then the user interface may further indicate, for example via a pop-up or an indicator such as a by visual means, that the container is now ready for further processing, and/or that the container has already been automatically processed for further analysis in the test system, for example by indicating that the container is being transported to a centrifuge and subsequent centrifugation. When the clotting wait time for the blood sample in the container has elapsed, the blood sample may be automatically transported to the centrifuge, so that the clot may be separated from the serum. The user interface may help the user to get an overview of and/or monitor samples that are still sitting in the storage/input section and are waiting for their individual clotting wait time to elapse, so they can be transferred to a centrifuge.

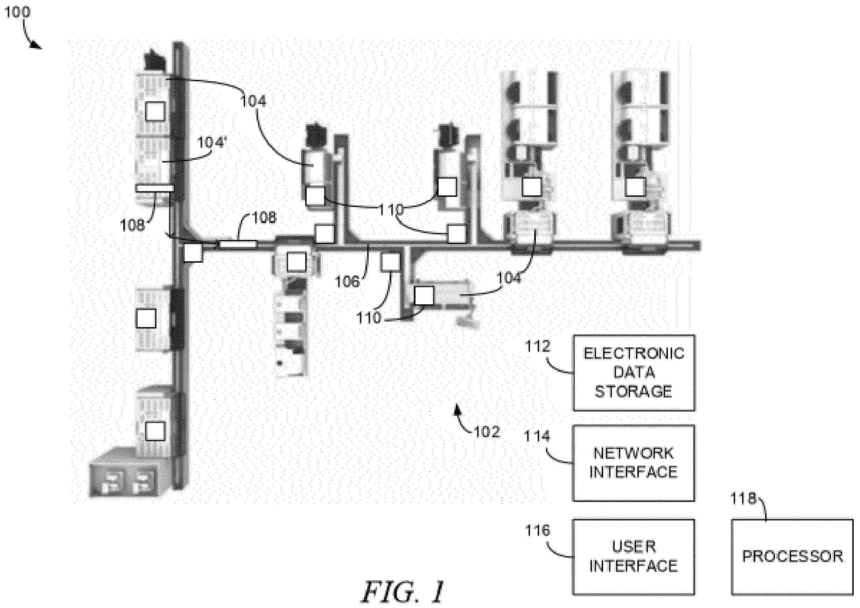

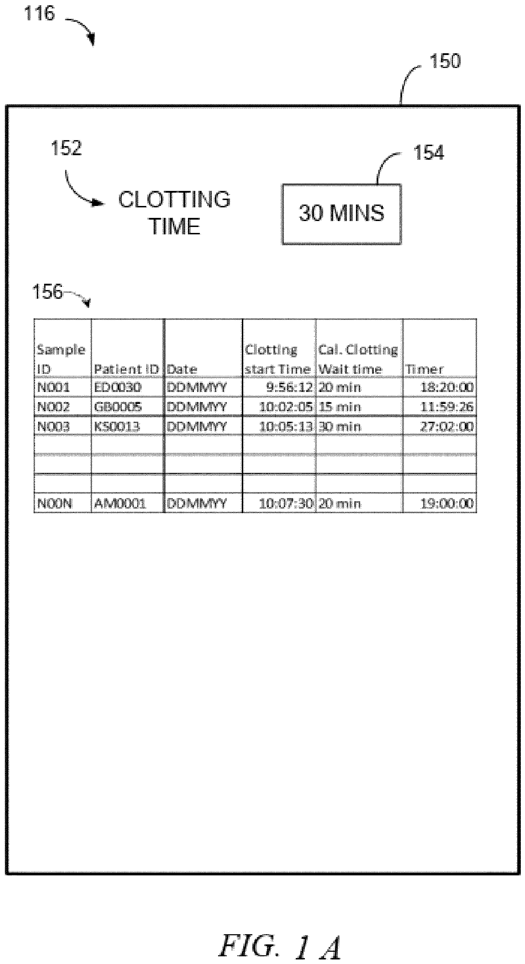

A coagulation agent may be added to the blood sample to accelerate the clotting process for the blood sample, which may be preferably done at the time of withdrawal of the blood sample. For example, snake venom may be used as a clotting agent to accelerate clotting of the blood sample to occur within a period of 2 to 5 minutes. In another example, thrombin may be used as a clotting agent and may result in accelerating clotting of the blood sample to occur in approximately 5 minutes. In another example, glass or silica particles may be used as a clotting agent for clotting the blood sample, which may accelerate the clotting process to occur in a period of 15 to 30 minutes. Addition of such a clotting agent may be taken into account for computing the clotting wait time, if this information is available for the test system, for example by reading the sample bar code on the sample container, which may also include information about the addition of coagulation agents to the blood sample. Therefore, accordingly, setting the clotting wait time for each sample may be based on certain pre-defined factors that may be prevalent or used or determined from historical facts. For example, determination of any factors from historical facts can be obtained from the patients past history or from a patients electronic medical records that may be accessed by the clinician. For example, pre-determined factors may include temperature, pressure and/or humidity during the transport and/or storage of the blood sample, wherein all of these factors may influence the clotting time. Accordingly the user interface may be used to display and/or modify the clotting wait time based on such pre-determined factors. These pre-determined factors are discussed hereinabove and hereinbelow.FIG. 1 illustrates an exemplary embodiment of test system (hereinafter also referred to as laboratory device or laboratory system) 100, wherein biological samples, such as blood samples, e.g. collected from patients may be tested. Test system 100 may specifically be a serum analysis unit, but not limited to a serum analysis unit. Test system 100 includes an automated sample test track 102, which further includes a plurality of pre-analytical units (like a sample input or a centrifuge), post-analytical units (like a sample output or a sample storage) and automated test stations 104 (also referred to as analyzers) coupled by conveyance unit 106. Conveyance unit 106 is configured to allow for container (also referred to as tubes) conveyance via for example a conveyor belt or for the movement of individually propelled container carriers within the test system.

Automated test stations 104 as illustrated herein may include various capabilities depending on the type of samples to be tested and the type of analytical tests to be performed. Conveyance system 106 may be or include a conveyor belt, a designated container carrier movement area and/or robotic arms, and is specifically designed to allow for the movement of sample containers 108 within the test system. based on the tests ordered for the blood sample, to at least one test station 104, which is either connected to the test-station or to a sample output location, from which samples can be transferred to standalone analyzers, which are not connected to conveyor system 106 of the test system.. Scheduling algorithms and routing algorithms may be used to efficiently route sample containers within the automated test station

Each automated serum test station 104 can utilize a certain amount of blood serum, contained in the sample container, in order to perform the requested sample analysis. Generally a test station can use a pipettor device to either aspirate the required serum sample volume directly from a position in close proximity to conveyor system 106 path, or from a position with the test station, to which the serum sample has been transferred to.

Sample containers may also be broadly referred to as vials or tubes, and are configured to contain blood samples and ultimately also the serum that will be provided to the analyzers for performing specific analysis as ordered.

The automated test station 104' may also include a clotting station, where samples may be placed in order to await the clotting reaction to occur. The clotting station may be spatially separated from, but connected to the automated test stations via conveyor system 106.The clotting station may be a dedicated storage module or an area or rack within a sample input module of the test system, where a blood sample can be stored after it's clotting wait time has been assigned, until the clotting time has elapsed. The clotting wait time may be determined or calculated as described previously.

The clotting station includes a blood storage unit that is configured to secure and maintain containers 108 that contain the blood samples. The blood samples are allowed to clot at the clotting station, thereby developing a serum and a clot fraction in container 108. Generally, a clotting wait time is assigned to samples stored at the clotting station. On positive determination of the clotting waiting time to have elapsed, the blood clotting reaction is considered to have occurred and the sample container can then be transported to a centrifuge in order to separate the clots from the serum, which is to be used by the test stations for further analysis.

The blood storage unit can accommodate one or more test tube holders, also referred to herein as racks, that is typically configured to maintain containers 108 in a vertical orientation with a lid or stopper positioned at the top of the container. The container is ideally maintained in a basically vertical position with the lid on the top, so that proper clotting of the blood sample occurs, and there is no clotting in and around the lid.

A sample container may be encoded or stamped with a clotting start time before being provided to the clotting station. The clotting station maintains containers 108 for a clotting wait time, allowing the blood samples to clot. The samples may be stored at the clotting station at room temperature or at different temperatures. A clotting wait time can be determined and adjusted according to factors such as temperature, pressure and humidity at the clotting station, and then used by test system 100 in order to ensure a complete clotting process. A timer is be used to monitor the clotting wait time and to determine when the clotting wait time has elapsed. When the clotting wait time has elapsed, the blood sample is provided to a centrifuge for centrifugation.

The process of centrifugation separates the residual clot and the serum. The centrifuged sample container is then either transported to at least one test station for further analysis or an aliquot is taken from the sample contained and transferred into a new sample tube, which is transported to at least one test station for further analysis which needs to be performed on the sample.

Generally, each sample container can include a unique sample identifier (not illustrated in the figure) or sample tag that identifies at least one of the patient, the sample type and the tests ordered for the specific sample. The unique identifier may also contain additional data such as the date and/or time of the sample withdrawal, information regarding the addition of coagulation agents or anticoagulation agents, patient specific information related to person health information, and other information that may be of relevance for calculating the appropriate length of the coagulation reaction. The unique identifier may further include data related to the healthcare provider that obtained the sample, a destination for the tests results and also an indication of one or more analytical tests that need to be performed on the blood sample by the test system. Subject to the list of requested tests, the sample may be routed to an appropriate test stations 104 of test system 100. The time stamp on unique identifier of the container may indicate the clotting start time. Alternatively, the clotting start time may be set when a sample container is first received by the test system and the relevant sample information is obtained by reading the unique sample identifier. The clotting start time may be automatically determined and the clotting wait time may be automatically set by the test system. The test system may be configured to monitor the clotting wait time for at least one of a plurality of samples, such that when the clotting wait time elapses for a specific sample, the test system may be configured to automatically transport the sample to a centrifuge for centrifugation of the blood sample and to begin further processing of the blood sample as appropriate.

Sample containers may include unique sample identifiers such as a 2D or 3D barcode, a radio frequency identification ("RFID") tag, or the like. Automated sample test track 102 includes various readers 110, for example identity or identification readers, positioned throughout test system 100.

Test system 100 further includes electronic data storage 112, network interface 114, user interface 116, and processor 118. Components 112, 114, 116, 118 may be local to test system 100 or may be accessed remotely by test system 100. Network interface 114 communicatively couples readers 110, electronic data storage 112, user interface 116, processor 118 together. Electronic data storage 112 stores records, such as electronic medical records ("EMRs"), which in addition to patient details may also contain information relating to containers that contain other information/data related to a patient within test system 100 and the samples contained therein. For example an EMR may be an electronic repository or a database or a data storage, which may store data either in a structured format or an unstructured format or a combination thereof. Electronic data storage 112 may further include registers, buffers, and the like for the operation of automated sample test track 102 including operation of the test system. Processor 118 provides general processing for test system 100. The test system may be connected by means of a wired network or a wireless network or a combination thereof.

Generally, based on the patient history available via an EMR, a clotting agent may be added to the blood sample by the clinician to accelerate the clotting process. The test system may be completed automated to identify the type of clotting agent to be added to a patients' blood sample and chose an appropriate clotting agent and add the clotting agent to the container containing the blood sample. For example, if a patient's EMR indicates that the patient is on anti-coagulation medication, an appropriate clotting agent may be added to the blood sample to accelerate the clotting process at the clotting station or alternatively the clotting wait time may be increased manually by means of user interface 116 coupled to the test system. Further processing of the blood sample occurs only after the clotting wait time has elapsed. In another example, if a patient's EMR indicated that the patients' blood clots very quickly, the clotting wait time for the blood sample can be reduced either automatically by the system or manually by means of a user interface 116, before further processing of the blood sample. In another example, it may be sometime beneficial to use a shorter clotting wait time for sample that may need to be processed on an urgent basis, when a patient is in an intensive care unit. In such a case ideally the clotting wait time is narrowed down to the best extent possible and reduce the clotting wait time to the shortest possible time, such that faster processing of the blood sample is possible in order to obtain the results as the earliest possible convenience. Alternatively, on the other hand it will be necessary to ensure and avoid malfunctioning of analysers due to non-fully clotted blood samples which may result in obstructing the pipetting device of the analyzers, leading to possibly incorrect liquid metering or contamination of the pipettor etc. Therefore ideally, use of a clotting wait time that is neither too short not too long will ensure proper and faster processing of the blood sample.

FIG. 1A illustrates an exemplary embodiment of user interface 116 for managing clotting within automated sample test track 102 or test system 100, which may include at least one serum analyzer. User interface 116 may be display screen 200 on a computer display or on a display for a tablet computer, smartphone, or the like, wherein information may be input by a user for the test system and output from the test system to be displayed to a user, via user interface 116. User interface 116 may have or could be a touchscreen interface and/or may be interfaced with via a mouse or other remote device.

User interface 116 may display clotting wait time 152 for at least one sample container 108. In various examples, clotting wait time 152 may be input or altered by a user/clinician via user interface 116, e.g. by one or more user inputs, or may be automatically determined by processor 118, for example based on the manufacturer's standards. Clotting wait time 152 may be monitored by timer 154, in an example this could be a countdown timer, starting at the clotting time and counting down until zero, at which time a blood sample in the container 108 would be expected to have completed the clotting process and clotted. In a more realistic situation there would be multiple samples sitting in the clotting storage/rack and many individual timers are running down as illustrated in table 156. The table records information on the sample ID which may be assigned by the laboratory. The patient ID may be provided so as to refer the HER and obtain additional information regarding the patients' history information, which may provide insight into any anti-coagulation treatment/medication that the patient may be undergoing. The clotting start time is noted, which as mentioned previously may the time or withdrawal of the blood sample or the time the blood sample container is loaded to the test system or the sample receipt time as recorded. The clotting wait time is calculated and indicated for the lab technician, which may be changed by the lab technician depending on for example, whether a clotting agent has been added which may accelerate the clotting process. A timer is also indicated which illustrates the time left for the clotting wait time to elapse after which the sample may be sent to the centrifuge. While a countdown timer is described, it is to be recognized and understood that a timer may instead count up to the clotting wait time 152, and on completion of the count-up to the clotting wait time, provide the container for centrifugation and subsequent further analysis to the test system. The timer may also be shown separately from clotting wait time 152, with the clotting wait time displayed while the timer counts separately. There could be multiple timers set of multiple containers as illustrated in the table in exemplary form, and can for example be maintained by an internal clock system of the processor of the test system. Further, the display may be configured to also provide a table of the sample ID and the time per sample, when a plurality of sample (not shown in the figure) exists.

Upon receiving container 108, which for example may be based on a notification from reader 110 in pre-analytical test station 104', processor 118 may determine a clotting start time for the clotting process. In various examples, the clotting start time for the timer is based on the record corresponding to container 108 and the time logged at which reader 110 identifies container 108. Alternatively, the test system may be configured to store a sample receipt time, determined via the reader 110, or a blood withdrawal time as the clotting start time in the electronic data storage 112 prior to transferring the receipt time to processor 118. Processor 118 may access the receipt time from the distribution buffer and base subsequent determinations around the timer on the receipt time from the distribution buffer.

Processor 118 may obtain the clotting wait time from a user via the user interface 116 or may determine the clotting wait time based on various factors, as has been disclosed previously and below. The speed of the blood clotting reaction may for example dependent on environmental conditions and the properties of the blood sample. The clotting of blood in a standard sample tube may normally be expected to occur within thirty (30) minutes when the ambient temperature is preferably at room temperature (i.e., twenty-five (25) degrees Celsius). However, an ambient temperature that is less than room temperature may tend to delay clotting, while an ambient temperature higher than room temperature may tend to accelerate clotting. Additional factors, such as the introduction of a clotting activator or clotting accelerator or clotting agent, such as snake venom/thrombin or glass or silica particles, may be used to predictably reduce clotting time, anywhere from two minutes to thirty minutes depending on the clotting agent used.

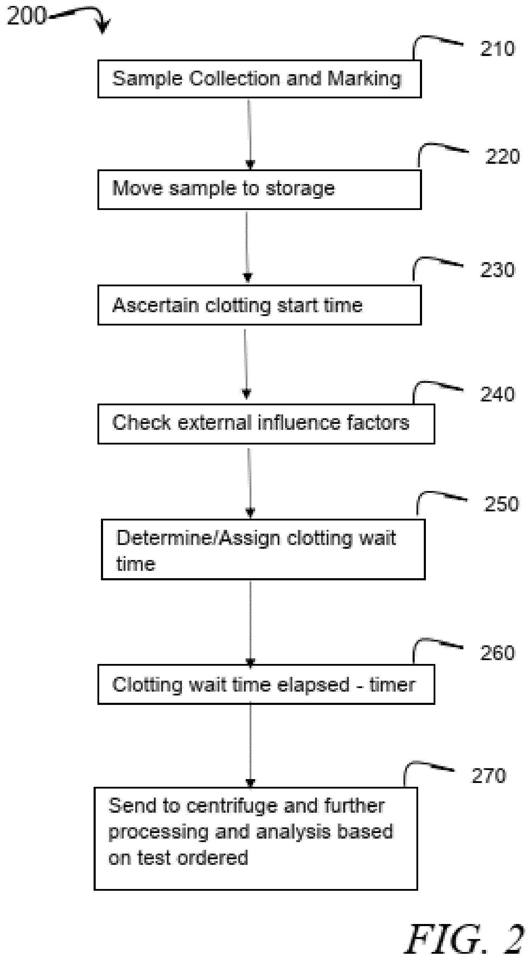

FIG. 2 is an exemplary embodiment of a method for processing blood samples 200 in accordance with the present disclosure. It should be understood that FIG 2. depicts an exemplary flow chart to illustrate a process that is considered to fall under the present disclosure. The process illustrated here is typically from the withdrawal of a blood sample to clotting and further processing of the blood sample in very generic terms.

A blood sample is first collected in a container from a patient and then encoded with the patient ID and other relevant parameters on an identifier attached to the container, optionally also including the date and time of withdrawal of the blood sample (Step 210). Other parameters may be encoded on the identifier as well. Readers positioned in a test system or a laboratory device may be configured to read the identifier and interpret the information encoded on the identifier. These identifiers are attached to or are otherwise associated with the sample container. For example, the identifier may contain the tests ordered for a patient, and the test system may be configured to route the sample to the specific analyzers or specific test stations to perform the particular tests ordered. As discussed previously, this data may be mapped and stored in a server interfaced with the test system or in an electronic storage of the test system. The time of recorded sample receipt within the test system or the time assigned as the time of withdrawal of the blood sample on the identifier may be identified as the clotting start time (Step 220).

Once the sample is collected in the container and marked with relevant details/information on the identifier, the sample is input onto the test system, normally at a sample input module of the test system and may then either stay at the input module for the duration of the clotting wait time or it may be moved to a dedicated storage/clotting area (Step 230) to allow the sample to undergo a complete clotting reaction. The clotting start time can be determined by readers positioned on the test system, and transmitted to a processor of the test system, based on either the sample draw time or based on the time when the sample was received at the test system. A user may be allowed to individually modify the clotting start time if needed. There could be multiple containers having the same clotting start time or there could be multiple containers having different clotting start times. Likewise, different clotting wait times may be assigned to the various samples because for each sample different factors which are of relevance for the clotting reaction, as discussed previously, may apply. The test system is adapted to monitor the clotting wait time assigned for each sample as illustrated in an exemplary format in Table 156 of FIG 1A.

Before moving the blood sample to the storage facility, where clotting occurs, a check can be made to ascertain whether there are any external conditions or factors that may influence the clotting of the blood sample in the container (Step 240). For example, temperature of the storage may influence the clotting wait time for the blood sample. In another example, blood sample from a patient on anti-coagulant medication may influence the clotting time. There could be several other factors that may influence the clotting wait time, and these have been disclosed previously. If it is determined that there are any factors influencing the clotting of the blood, then a clinician/technician may ensure that proper clotting has occurred before the sample is out for further processing. For example, one such measures may include adding a clotting agent to accelerate the clotting process or the clotting wait time may be increased or decreased by means of a user interface provided on the test system. In another example, when it may be required to obtain an urgent analysis on the blood sample of a patient in the intensive care unit, where the clotting wait time may be tailored to be shorter to obtain results faster.

The Clotting wait time may be computed or processed taking into account any other conditions influencing the clotting process for the blood sample (Step 250). If there are no factors influencing the clotting of the blood sample, then a default clotting wait time is assigned to the sample, which is preferably about 30 mins., and a timer may be assigned to monitor the clotting wait time. The processor of the test system or laboratory device may be configured to automatically allocate a timer to monitor the clotting wait time or alternatively a clock may be set by the test system to monitor the clotting wait time. Manual modification of the clotting wait time automatically assigned by the test system may be performed via the user interface of the test system by a user of the test system. The clotting wait time may be counted either in a descending manner or an ascending manner, as convenient by the user. If there are external factor influencing the clotting wait time, then the clotting wait time may be either increased or decreased manually by means of an input via the user interface, wherein the clotting wait time can be reset manually. For example, if the patients EMR indicates that the patient is on anti-coagulants medication, then either a clotting agent may be added to accelerate the clotting process or the clotting wait time may be substantially increased by the user to allow the blood sample sufficient time for clotting.

On positive determination that the clotting wait time has elapsed for the blood sample (Step 260), the blood sample is then automatically sent for further processing to a centrifuge for centrifugation, to separate the serum and the clot. Post centrifugation, the serum is then sent to test stations or analyzers for processing based on the test order, as described previously with respect to FIG. 1.

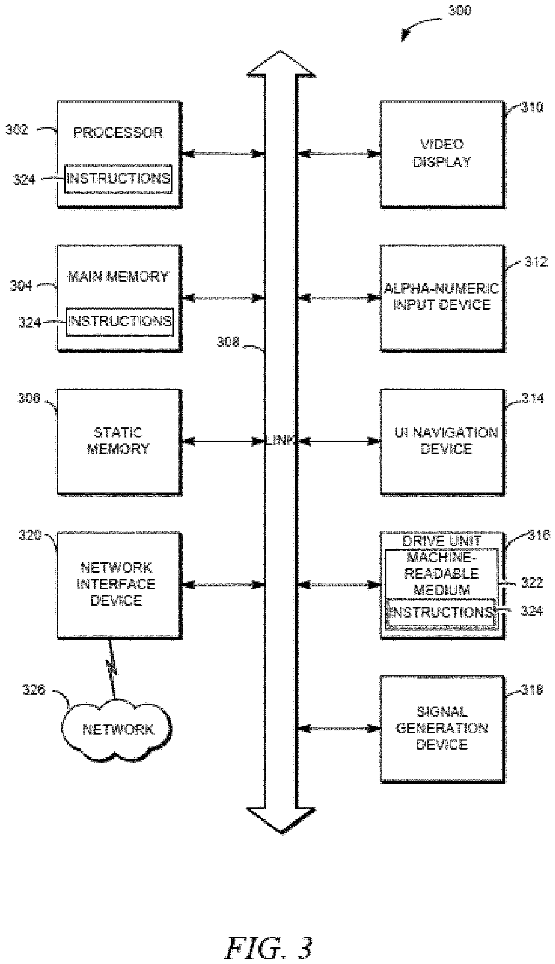

FIG. 3 illustrates an exemplary embodiment of a block diagram components of machine 300, the machine configured to be able to read instructions from a machine-readable medium, for example a machine-readable storage medium, and perform any one or more of the methodologies discussed herein. Specifically, in accordance with the embodiments of the present disclosure, FIG. 3 illustrates a diagrammatic representation of machine 300 in the example form of a computer system, that may be externally attached to the test system or may be built in as part of the test system itself that may be configured to drive the test system and the entire process of blood sample analysis, from reading the clotting start time that is assigned, to computing the clotting wait time, to monitoring the clotting wait time to processing the sample at the various analysers of the test system. The example computer system includes instructions 324, software in the form of code or binary executables or bytecode or scripts etc., causing machine 300 to perform any one or more of the methodologies/techniques discussed herein, when the instruction is implemented on the machine. In an alternative example, machine 300 may operate as a standalone device or may be connected, for example via a network, to other machines. In a networked deployment, machine 300 may be configured to operate in a capacity as a server machine or a client machine in a server-client network environment, or as a peer machine in a peer-to-peer or distributed network environment.

Machine 300 may be or include a server computer, a client computer, a personal computer (PC), a tablet computer, a laptop computer, a netbook, a set-top box (STB), a personal digital assistant (PDA), a cellular telephone, a smartphone, a web appliance, a network router, a network switch, a network bridge, or any machine capable of executing instructions 324, sequentially or otherwise, that specify actions to be taken by that machine. Further, while only a single machine is illustrated, the term "machine" shall also be taken to include a collection of machines that may individually or jointly implement instructions 324 to perform any one or more of the methodologies discussed herein.

Machine 300 includes a controller, control circuitry and/or processor 302, e.g. a central processing unit (CPU). The control circuitry 302 may be configured to set and/or determine the clotting time; to set and/or determine the clotting wait time; and to automatically provide the container and/or blood sample to the centrifuge upon determination of completion of the clotting wait time.

Machine 300 further includes a graphics processing unit (GPU), a digital signal processor (DSP), an application specific integrated circuit (ASIC), a radio-frequency integrated circuit (RFIC), or any suitable combination thereof, main memory 304, and static memory 306, which are generally configured to communicate with each other via bus 308. Machine 300 may further include graphics display 310 (a plasma display panel (PDP), a light emitting diode (LED) display, a liquid crystal display (LCD), a projector, or a cathode ray tube (CRT)). Machine 300 may also include alphanumeric input device 312 (a keyboard), cursor control device 314 ( a mouse, a touchpad, a trackball, a joystick, a motion sensor, or other pointing instrument), storage unit 316, signal generation device 318 (a speaker), and network interface device 320.

Storage unit 316 includes machine-readable medium 322 on which is stored instructions 324 (software, as defined previously) embodying any one or more of the methodologies or functions described herein. Instructions 324 may also reside, completely or at least partially, within main memory 304, within processor 302 (within the processor's cache memory), or a combination thereof, during execution by machine 300. Accordingly, main memory 304 and processor 302, may be considered as machine-readable media. Instructions 324 may be transmitted or received over network 326 via network interface device 320. The network interface device may be configured to be a wired device or a wireless device. Machine 300 may connect to other devices via wired means, wireless means or a combination thereof.

As used herein, the term "memory" refers to a machine-readable medium able to store data temporarily or permanently and may be taken to include, but not be limited to, random-access memory (RAM), read-only memory (ROM), buffer memory, flash memory, and cache memory. While machine-readable medium 322 is shown in an example to be a single medium, the term "machine-readable medium" should be taken to include a single medium or multiple media. For example a centralized or distributed database, or associated caches and servers, may be able to store instructions. The term "machine-readable medium" shall also be taken to include any medium, or combination of multiple media, that is capable of storing or carrying instructions, for example software, for implementation by machine 300, such that the instructions, when implemented by one or more processors of the machine, for example processor 302, cause the machine to perform any one or more of the methodologies described herein. Accordingly, a "machine-readable medium" refers to a single storage apparatus or device, as well as "cloud-based" storage systems or storage networks that include multiple storage apparatus or devices. The term "machine-readable medium" shall accordingly be taken to include, but not be limited to, one or more data repositories in the form of a solid-state memory, an optical medium, a magnetic medium, or any suitable combination thereof. While referring to a cloud-based storage system, the system may be either one of a private cloud, a public cloud or a hybrid cloud.

Throughout this specification, plural instances may implement components, operations, or structures described as a single instance. Although individual operations of one or more methods are illustrated and described as separate operations, one or more of the individual operations may be performed concurrently, and there is no requirement that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

Certain embodiments are described herein as including logic or a number of components, modules, or mechanisms. Modules may constitute either software modules, for example code embodied on a machine-readable medium or in a transmission signa, or hardware modules. A "hardware module" is a tangible unit capable of performing certain operations and may be configured or arranged in a certain physical manner. For example, one or more computer systems, such as a standalone computer system, a client computer system, or a server computer system, or one or more hardware modules of a computer system, such as a processor or a group of processors, may be configured by software, such as an application or application portion, as a hardware module that operates to perform certain operations as described herein.

A hardware module may be implemented mechanically, electronically, or any suitable combination thereof. For example, a hardware module may include dedicated circuitry or logic that is permanently configured to perform certain operations. For example, a hardware module may be a special-purpose processor, such as a field programmable gate array (FPGA) or an ASIC. A hardware module may also include programmable logic or circuitry that is temporarily configured by software to perform certain operations. For example, a hardware module may include software encompassed within a general-purpose processor or other programmable processor. It will be appreciated that the decision to implement a hardware module mechanically, in dedicated and permanently configured circuitry, or in temporarily configured circuitry, for example configured by software, may be driven by cost and time considerations.

Accordingly, the phrase "hardware module" should be understood to encompass a tangible entity, be that an entity that is physically constructed, permanently configured, such as hardwired, or temporarily configured, such as programmed, to operate in a certain manner or to perform certain operations described herein. As used herein, "hardware-implemented module" refers to a hardware module. Considering embodiments in which hardware modules are temporarily configured, for example programmed, each of the hardware modules need not be configured or instantiated at any one instance in time. For example, where a hardware module comprises a general-purpose processor configured by software to become a special-purpose processor, the general-purpose processor may be configured as respectively different special-purpose processors, for example comprising different hardware modules, at different times. Software may accordingly configure a processor, for example, to constitute a particular hardware module at one instance of time and to constitute a different hardware module at a different instance of time.

Hardware modules can provide information to, and receive information from, other hardware modules. Accordingly, as described, hardware modules may be regarded as being communicatively coupled. Where multiple hardware modules exist contemporaneously, communications may be achieved through signal transmission, for example over appropriate circuits and buses, between or among two or more of the hardware modules. In instances where multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. Again, a further hardware module may then, at a later time, access the memory device to retrieve and process the stored output. Hardware modules may also initiate communications with input or output devices, and can operate on a resource, for example a collection of information.

The various operations of example methods described herein may be performed, at least partially, by one or more processors that are temporarily configured, for example by software, or permanently configured to perform the relevant operations. Whether temporarily or permanently configured, such processors may constitute processor-implemented modules that operate to perform one or more operations or functions described herein. As used herein, "processor-implemented module" refers to a hardware module implemented using one or more processors.

Similarly, the methods described herein may be at least partially processor-implemented, a processor being an example of hardware. For example, at least some of the operations of a method may be performed by one or more processors or processor-implemented modules. Moreover, the one or more processors may also operate to support performance of the relevant operations in a "cloud computing" environment or as a "software as a service" (SaaS). For example, at least some of the operations may be performed by a group of computers or a group of processors, with these operations being accessible via a network, such as the Internet, and via one or more appropriate interfaces, such as an application program interface (API).

The performance of certain operations may be distributed among the one or more processors, not only residing within a single machine, but deployed across a number of machines, which may be coupled via a network in a fail-safe manner. In some example embodiments, the one or more processors or processor-implemented modules may be located in a single geographic location, for example within a home environment, an office environment, a hospital or a clinic or a laboratory or a server farm. In other example embodiments, the one or more processors or processor-implemented modules may be distributed across a number of geographic locations.

Some portions of this specification are presented in terms of algorithms or symbolic representations of operations on data stored as bits or binary digital signals within a machine memory, such as a computer memory. These algorithms or symbolic representations are examples of techniques used by those of ordinary skill in the data processing arts to convey the substance of their work to others skilled in the art. As used herein, an "algorithm" is a self-consistent sequence of operations or similar processing leading to a desired result. In this context, algorithms and operations involve physical manipulation of physical quantities. Typically, but not necessarily, such quantities may take the form of electrical, magnetic, or optical signals capable of being stored, accessed, transferred, combined, compared, or otherwise manipulated by a machine. It is convenient at times, principally for reasons of common usage, to refer to such signals using words such as "data," "content," "bits," "values," "elements," "symbols," "characters," "terms," "numbers," "numerals," or the like. These words, however, are merely convenient labels and are to be associated with appropriate physical quantities.

Unless specifically stated otherwise, discussions herein using words such as "processing," "computing," "calculating," "determining," "presenting," "displaying," or the like may refer to actions or processes of a machine, for example a computer, that manipulates or transforms data represented as physical, such as electronic, magnetic, or optical, quantities within one or more memories, for example a volatile memory, a non-volatile memory, or any suitable combination thereof, registers, or other machine components that receive, store, transmit, or display information. Furthermore, unless specifically stated otherwise, the terms "a" or "an" are herein used, as is common in patent documents, to include one or more than one instance. Finally, as used herein, the conjunction "or" refers to a non-exclusive "or," unless specifically stated otherwise.

## Claims

1. A method for processing a blood sample contained in a container (108) in a test system (100), the method comprising:
a. setting a clotting start time for the blood sample and/or the container (108);
b. setting a clotting wait time (152) to allow the blood sample to clot; and
c. on positive determination of completion of the clotting wait time (152), automatically providing the blood sample to a centrifuge coupled to the test system (100).

2. The method as claimed in claim 1, wherein the clotting start time is set as at least one of:
i. a time of withdrawal of the blood sample into the container (108); and
ii. a time of loading the container (108) containing the blood sample onto the test system (100).

3. The method as claimed in any of the preceding claims, wherein the clotting wait time is determined based on at least one of:
b. a time of withdrawal of the blood sample into the container (108);
c. a time of loading the blood sample in the container (108) onto the test system (100);
d. addition of at least one clotting agent to the blood sample and/or the container (108);
e. a storage temperature of the blood sample;
f. a measure of an air pressure and/or a measure of humidity during a storage of the blood sample;
g. at least one patient specific coagulation parameter;
h. a predefined clotting wait time (152), optionally as specified by the test system manufacturer.

4. The method as claimed in claim 3, wherein the at least one patient specific coagulation parameter is derived from at least one of an electronic medical record (EMR), a Laboratory Information System (LIS), a Hospital Information System (HIS), and any other Personal Health Information (PHI).

5. The method as claimed in any of the preceding claims, further comprising monitoring the clotting wait time with a timer (154)of the test system (100).

6. The method as claimed in any of the preceding claims, further comprising
i. subjecting the container (108) comprising the blood sample to a centrifugation step to segregate a serum from a residual clot; and
j. providing the serum to at least one test station (104), the at least one test station being part of or connected to the test system (100), for further analysis, preferably wherein the test station (104) is a blood serum analyzer.

7. The method as claimed in claim 3, wherein at least one clotting agent is comprised in or added to the blood sample, which is configured to reduce the clotting wait time (152) for the blood sample.

8. The method as claimed in any of the preceding claims, wherein the clotting wait time (152) is entered, received and/or provided via a user interface (116) coupled to the test system (100).

9. The method as claimed in any of the preceding claims, wherein the clotting wait time (152) is selected, computed and/or determined automatically by the test system (100).

10. The method as claimed in any of the claims 3 to 9, wherein the clotting wait time (152) is adjusted to be either longer than or shorter than a predefined default clotting wait time based on the at least one patient specific coagulation parameter.

11. The method as claimed in any of the preceding claims, wherein the container (108) is held in a vertical position during the clotting wait time (152), wherein the top of the container (108) is either open or closed with a cap, a lid, a stopper, a film, or any other container closing structure.

12. The method as claimed in any of the preceding claims, wherein a time of the withdrawal of the blood sample into the container (108) is obtained from a sample related data source.

13. The method as claimed in claims 12, wherein the sample related data source is a unique identifier comprising at least one of a bar code, a MEMS, smart dust, an RFID chip or a NFC chip coupled to the container.

14. A laboratory test system (100) comprising:
k. a container storage for storing the at least one container (108) containing a blood sample for a duration of a clotting wait time (152);
I. an automated sample conveyance system (106) coupled to one or more test stations (100); and the conveyance system (106) configured to convey the at least one container (108) to at least one of the one or more test stations (100), and
wherein the test system (100) is configured to perform steps of the method as claimed in any of the preceding claims.

15. A computer-readable medium comprising instructions which, when executed by a processor of a computing device associated with a test system (100), causes the test system (100) to carry out steps of the method as claimed in any of claims 1 to 13.
